# EUROPEAN PATENT SPECIFICATION

(11) **EP 4 452 277 B1**
(45) Date of publication and mention of the grant of the patent: **10.12.2025**
(21) Application number: 22854172.8
(22) Date of filing: 19.12.2022
(51) Int. Cl.: A61K 31/685, A61P 25/24, A61P 25/28

(54) **INDUSTRIAL PROCESS FOR THE EXTRACTION AND PURIFICATION OF PHOSPHOLIPIDS**
INDUSTRIELLES VERFAHREN ZUR EXTRAKTION UND REINIGUNG VON PHOSPHOLIPIDEN
PROCÉDÉ INDUSTRIEL D'EXTRACTION ET DE PURIFICATION DE PHOSPHOLIPIDES

(30) Priority: 22.12.2021 IT 202100032252
(43) Date of publication of application: 30.10.2024
(73) Proprietor: Fidia Farmaceutici S.p.A., 35031 Abano Terme (PD) (IT)
(72) Inventor: PITTARELLO, Mara, 35031 Abano Terme (PD) (IT)
(74) Representative: Bird & Bird Società tra Avvocati S.r.l.
(86) International application number: PCT/IB2022/062486
(87) International publication number: WO 2023/119129

(56) References cited:
- EP-A1- 0 148 045
- WO-A1-93/21190
- CN-A- 1 583 766
- US-A- 3 436 413
- FOLCH J ET AL: "A SIMPLE METHOD FOR THE ISOLATION AND PURIFICATION OF TOTAL LIPIDES FROM ANIMAL TISSUES", JOURNAL OF BIOLOGICAL CHEMISTRY, AMERICAN SOCIETY FOR BIOCHEMISTRY AND MOLECULAR BIOLOGY, US, vol. 224, 1 January 1957 (1957-01-01), pages 497 - 509, XP009011060, ISSN: 0021-9258
- BIGGIO GIOVANNI ET AL: "Overview of the pharmacological properties and therapeutic efficacy of phospholipid liposomes (Liposom Forte ) in patients with depressive disorders", vol. 59, no. 1, 1 March 2018 (2018-03-01), IT, pages 45 - 53, XP055944728, ISSN: 0391-1772, Retrieved from the Internet <URL:https://www.minervamedica.it/en/journals/minerva-psychiatry/article.php?cod=R17Y2018N01A0045> DOI: 10.23736/S0391-1772.17.01955-0

## Description

### OBJECT OF THE INVENTION

The present invention describes and claims a new industrial process for the extraction and purification of phospholipids from animal brain, particularly pure and with a high percentage content of phosphatidylserine (PS) and phosphatidylcholine (PC), especially enriched in PS, and the use of said phospholipids in the treatment of the pathology defined as "Long-Covid".

### FIELD OF THE INVENTION

Phospholipids represent a class of lipids that contain phosphate, they are amphipathic molecules as they have a hydrophilic polar head and a hydrophobic non-polar tail, and are the main constituents of animal and plant cell membranes.

They can generally be divided into sphingophospholipids and glycerophospholipids (or phosphoglycerides), the latter deriving from sn-glycerol-3-phosphate wherein the glycerol (CH₂OH-CHOH-CH₂OH) is esterified in position 3 with orthophosphoric acid (H₃PO₄) and in position 2 with a fatty acid, whereas different classes of compounds can be bound in position 1.

There is also the class of diacyl-phospholipids when the glycerol is esterified in position 1 and in position 2 with fatty acids and in position 3 with orthophosphoric acid.

In membrane phospholipids, there are two types of fatty acids: saturated fatty acids in which all the carbon atoms are saturated, and unsaturated fatty acids in which one or more double bonds are present; the fatty acid in position 2 of the diacyl-phosphoglycerides of cell membranes is usually unsaturated.

In addition to esterification with glycerol (which leads to the formation of phosphatidic acid), orthophosphoric acid has a second esterification with an alcohol (an amino alcohol or an amino acid with an alcohol group or a sugar), diacylphospholipids are therefore indicated with the prefix phosphatidyl - followed by the name of the esterified compound with the phosphate group (for example phosphatidyl serine wherein serine is the esterified compound with the phosphate group).

Phospholipids represent about 70-80% of the lipid weight of cell membranes, and the main ones are: phosphatidylcholine, phosphatidylserine, phosphatidylethanolamine and sphingomyelin, which together form over 50% of all membrane lipids. Phosphatidylinositol, on the other hand, is a phospholipid present in smaller quantities which however plays a crucial role in the genesis of intracellular signals.

Sphingophospholipids contain a long-chain aminoalcohol instead of glycerol: in sphingosine (C18) the amino group is bound with an amide bond to the carboxyl group of a fatty acid (forming a compound called ceramide), whereas the hydroxyl group is bound with an ester bond to the orthophosphate, in turn esterified with an amino alcohol, usually choline, thus forming a compound called sphingomyelin or ceramide-1-phosphorylcholine; when the ceramide, on the other hand, is bound via the hydroxyl group to a monosaccharide, a cerebroside is obtained, if the ceramide is bound via the hydroxyl group to an oligosaccharide, a ganglioside is formed, both gligolipids being phosphorus-free.

Phosphatidylcholine PC is the major phospholipid of eukaryotic cells comprising 40 to 50% of membrane phospholipids: an important molecule for cell proliferation and division, it represents the main source of choline for cholinergic neurons which use it in the synthesis of the neurotransmitter acetylcholine; when the demand for choline exceeds the re-synthesis turnover of phosphatidylcholine, the composition of the neuronal membranes can undergo changes which are such as to negatively affect their integrity and therefore the vitality of the neurons involved. Acetylcholine is the neurotransmitter that mediates important neuronal functions such as respiration, muscle contraction, heart rhythm, short-term memory, and most of the pre-ganglionic neurons of the Sympathetic and Parasympathetic system, as well as motor neurons, use this neurotransmitter.

Phosphatidylethanolamine (PE) represents from 20 to 50% of the membrane phospholipids of mammals, but it represents about 30-45% of brain phospholipids, whereas phosphatidylserine is, on the contrary, one of the phospholipids with the lowest concentration, present for only 2-10% of total phospholipids (Vance JE.; Journal of Lipid Research; 2008; 49:1377-1387). Phosphatidylethanolamine is found particularly in the white matter of cerebral nervous tissue, nerves and spinal cord, where it participates in membrane fusion during cell division cytokinesis; PE is also an important precursor/substrate in numerous and various biochemical and cellular physiological processes in mammals.

Phosphatidylserine PS is the main acid membrane phospholipid, capable of influencing the stability, fluidity and organization of cell membranes, it is mainly distributed in the internal part of plasma membranes where it interacts with cytoplasmic elements, proving to be capable of activating two important cellular enzymes such as sodium/potassium *ATPase* and *protein kinase C.*

Phosphatidylserine is perhaps the most widely-studied phospholipid due to its demonstrated capacity of both stimulating the release of dopamine from the dopaminergic terminals of the striatum, and activating the adenylate-cyclase enzyme of hypothalamic neurons, but above all for preventing the loss of dendritic spines of hippocampal pyramidal neurons linked to brain aging (Advances in Behavioural Biology; Lecithin Ed. by Hanin I. and Ansell GB.; vol.33; 1987).

For all of the reasons listed above, PS has been the subject of numerous clinical trials in the evaluation of the prevention and/or therapy of cognitive decline in elderly persons and/or Alzheimer patients and depressive disorders in general (Cenacchi T. et al.; Aging Clin Exp Res; 1993; 5:123-133; Biggio G. et al. Minerva Psichiatrica; 2018; 59(1):1-10). It has also been demonstrated how phosphatidylserine can counteract the increase in the cortisol hormone in subjects subjected to stress, facilitating their functional recovery (Monteleone P. et al.; Neuroendocrinology; 1990; 52(3):243-8).

Animal phospholipids are normally extracted from the membranes using solvents or mixtures of solvent, among which, in particular, chloroform, a recognized carcinogenic solvent, with subsequent purification by means of silica gel chromatography (SU1,102,603; EP638083; US20120116104;); this solvent is also widely used in the purification of gangliosides from which the phospholipids are eliminated by chloroform-methanol partitioning (EP0150712), or by saponification (EP3095451).

Further, CN 1 583 766 A discloses a process of extraction of PS from brain tissue. The process comprises the steps of: (1) grinding of brain tissue, addition of acetone, stirring & settling, isolation of PL extract; (2) addition of hexane, ethanol or ammonia/ethanol, isolation of PL extract, washing with an organic solvent chosen from ether, hexane, toluene, CH₃CI, chloroform & filtration, washing with Na₂CO₃ at pH=8.5, freeze-thawing, acidification with HCI pH=5-6, evaporation obtain a dry powder; (3) addition of hexane, addition of Et0H/sodium acetate, isolation and evaporation to produce PS in 53%-93%% purity. WO 93/21190 A1 concerns the preparation of a phospholipid mixture comprising PS from bovine brain which is free from pathogenic agents. The process comprises an extraction process and a purification method which is chosen from organic extraction or column chromatography. US 3 436 413 also relates to the extraction of PL, in particular PS, from brain tissue. The article of Folch J. et al: "A simple method for the isolation and purification of total lipides from animal tissues" (Journal of Biological Chemistry, American Society for Biochemistry and Molecular Biology, US, vol. 224, 1 January 1957 (1957-01-01), pages 497-509) examines extraction methods of lipids from animal tissue. The process comprises an extraction and washing steps and, in particular, it is examined the influence of the salts used on the distribution of the lipids in the different phases. The review article of Biggio Giovanni et al.: "Overview of the pharmacological properties and therapeutic efficacy of phospholipid liposomes (Liposom Forte) in patients with depressive disorders" (MINERVA PSICHIATRICA, vol. 59, no. 1, 1 March 2018 (2018-03-01), pages 45-53) is a review article on the pharmaceutical properties and clinical trials of PL for treating depressive disorders. EP 0 148 045 A1 relates to a phospholipid composition for treating CNS disorders, in particular disorders related to aging of brain.

The need is therefore felt for an industrial process for the extraction and purification of the above-mentioned phospholipids from animal brain tissue in which solvents such as chloroform are not used, to reach a final phospholipid product free of solvent residues, protein components and contaminants such as viruses in particular, and which is easy to implement as it does not require the use of chromatographic processes with silica gel.

The object of the present invention is to identify a process for the extraction and purification of animal phospholipids which overcomes the drawbacks of the state of the art, satisfies all the requirements listed above, and above all leads to the preparation of final PS-enriched phospholipids.

The present patent therefore relates to an innovative process for the extraction and purification of animal phospholipids as claimed in claim 1.

This process satisfies all the requirements listed above and, above all, leads to the preparation of final phospholipids enriched in PS, as the final PS/PC ratio obtained at the end of the extraction and purification steps of the phospholipids object of the invention is within the range of 0.8-1,6, thus being radically modified with respect to the initial PS/PC ratio of the original membranes of the animal tissues from which these phospholipids are extracted and purified, as previously defined.

With this innovative process, the Applicant has at its disposal a more effective phospholipid preparation in the treatment of all pathological conditions that require the administration of high concentrations of PS/PC, in particular PS, such as the prevention/treatment of problems of a neuroendocrine origin such as, for example, dementia and/or cognitive impairment in the elderly, and depressive disorders in general.

The present invention therefore further relates to phospholipids for use in the treatment of the pathology defined as "Long-Covid or Post-Covid", preferably phospholipids enriched in PS obtained according to the extraction and purification process object of the present invention for use in the treatment of the pathology defined as "Long-Covid or Post-Covid".

The disease Long-Covid affects a high percentage of patients who have recovered from the Sars-CoV-2 coronavirus and the pathologies caused by it, who unfortunately still show evident consequences of this infection; anosmia and ageusia, the persistent feeling of fatigue/asthenia (understood as the physical and/or mental decrease in the patient's performance), often headaches, sometimes serious psychiatric manifestations such as psychosis, are among the main neurological/neuroendocrine symptoms of Long-Covid, so-called "mental fog" is also frequently found, i.e., a state of mental confusion in which the patient who has recovered from the Sars-CoV-2 virus is unable to concentrate and have full control of his mental faculties, and therefore carry out the normal daily activities of his pre-Covid life (Walitt B. et al.; Pain Reports; 2021; 6(1) 887); finally, among the symptoms of the Long-Covid pathology various hormonal dysfunctions can also be found, that cause the lowering of the serum level of male testosterone, with a lowering of libido and associated consequent problems of erectile dysfunction (Moreno-Perez O. et al.; Clinical Endocrinology ; 2021; vol. 96; 3:353).

The use of phospholipids enriched in PS is therefore part of the treatment of the Long-Covid pathology for promoting/restoring the psycho-physical balance of the patient and reducing the symptoms of this pathology, above all improving mental fog, not only in adults but also in adolescents.

### DETAILED DESCRIPTION OF THE INVENTION

The Applicant claims an innovative industrial process for the extraction and purification of phospholipids from animal brain (preferably from the hypothalamic area and the cortical area), preferably swine, which does not contemplate the use of chloroform and a purification step by means of silica gel chromatography, which allows a particularly pure final phospholipid product to be obtained as it is not contaminated by unconventional animal viruses (above all from the viruses responsible for spongiform encephalopathy), free of solvent residues and protein components, with a high percentage content of PS and PC, but enriched above all in PS, wherein the final PS/PC ratio obtained at the end of the extraction and purification steps ranges from 0.8 to 1.6, thus proving to be radically modified with respect to the initial PS/PC ratio of the original animal tissue membranes from which these phospholipids are extracted and purified, as well as being different from the PS/PC ratio obtained using the extraction and purification methods known to the state of the art.

The phospholipid product obtained from the innovative process object of the invention consists of the phospholipids Phosphatidylethanolamine PE, Phosphatidic acid PA, Phosphatidylinositol PI, Phosphatidylserine PS, Phosphatidylcholine PC, Sphingomyelin SFM; the final product has only traces of two membrane glycosphingolipids such as Cerebroside (neutral glycolipid) and Sulfatide (glycolipid sulfate ester), also proving to be free of gangliosides and purified of any protein (and non-protein) contaminant and any solvent residue used.

They are disclosed also pharmaceutical compositions comprising or consisting of PS-enriched phospholipids obtained with the above industrial extraction and purification process of phospholipids from animal brain, and their use in the prevention/treatment of problems of a neuroendocrine origin such as, for example, dementia and/or cognitive impairment of the elderly, and depressive disorders in general.

In these pharmaceutical compositions, the PS-enriched phospholipids mentioned above can be associated with drugs and/or pharmacologically or biologically active agents such as, for example, steroids, non-steroidal antiinflammatory drugs, natural animal and/or vegetable extracts and/or vitamins, preferably vitamins of group B.

The invention further relates to phospholipids and the relative pharmaceutical compositions which comprise or consist of said phospholipids, for use in the treatment of the pathology defined as "Long-Covid".

Particularly preferred are the PS-enriched phospholipids obtained with the industrial process for the extraction and purification of phospholipids from animal brain object of the invention (as defined above) for use in the treatment of the pathology defined as "Long-Covid", as the Applicant has surprisingly discovered, and subsequently demonstrated, that treatment with these phospholipids significantly improves, and also resolves, the symptoms of Long-Covid patients.

The Applicant hereinafter describes and claims the process for the extraction and purification of phospholipids enriched in PS, wherein the final PS/phosphatidylcholine PC ratio at the end of the extraction and purification steps ranges from 0.8 to 1.6, which comprises or consists of the following essential steps: Extraction, which comprises the following steps:
a. grinding the animal brain;
b. adding a mixture of solvents comprising acetone and methanol to the ground product
c. stirring and settling;
d. separating the settled wet raw phospholipid extract;

Intermediate purification which comprises the following steps:
   e. solubilizing the wet raw phospholipid extract obtained from step d. with NaCl and KCl salts, settling, separating the subnatant by filtration;
   f. washing the subnatant obtained in the previous step e. with purified water, settling and separating the subnatant;
   g. adding acetone and CaCl₂ dihydrate to the subnatant of step f., stirring and settling;
   h. filtering the product of step g. by means of a filter press to obtain a first partially purified solid phospholipid extract;
Final purification which comprises the following steps:
   i. solubilizing the solid phospholipid extract in an organic solvent;
   j. adding a solution comprising EDTA, NaOH, KCl, and NaCl having a pH of 8.5-9 to the extract of step i., stirring and settling;
   k. separating the subnatant of step j., adding water and ethanol, stirring and settling;
   l. separating the subnatant of step k. by filtration through a filter with a filtration degree of 3µm, or alternatively 0.8 µm, and subsequently 0.2 µm, stirring and settling;
   m. washing the sediment from step l. with acetone;
   n. filtering the product of step m. using a filter press to obtain the purified solid phospholipid extract;
   o. treating the final extract of step n. with liquid nitrogen, granulating and vacuum-drying.

The Applicant hereinafter describes and claims the process for the extraction and purification of phospholipids enriched in PS, wherein the final PS/phosphatidylcholine PC ratio at the end of the extraction and purification steps ranges from 0.8 to 1.6, which comprises or consists of the following steps further detailed hereunder:
Extraction which comprises the following steps:
   wet raw phospholipid extracts are prepared with steps a-d:
   a. grinding the animal brain, preferably its hypothalamic part and/or its cortex;
   b. adding the solvent mixture consisting of acetone/methylene chloride or another polar organic solvent (POS)/methanol to the ground product of step a., in a ratio (Kg/L) (ground product) 1: (mixture) 1.5-3 w/v, preferably 1:2.2, w/v, wherein the solvents of the above mixture are in a ratio of (acetone)1:(methylene chloride or another POS)1-2: (methanol) 0.5-1 (v/v);
   c. leaving under stirring for at least 60 minutes at 30-35°C; settling the extract for at least 180 minutes at 30-35°C;
   d. separating the subnatant by filtering it through a filter with a filtration degree of 1µm, preferably in polypropylene, eliminating the supernatant;
Intermediate purification which comprises the following steps:
   with the following steps the phospholipid extract is enriched in PS through the use of CaCl₂ dihydrate which facilitates the sedimentation of PS, the phospholipid extracts are purified from any possible unconventional viruses and/or any pyrogens present in the initial animal derivative and, furthermore, the extract is also purified from proteins and gangliosides, as these molecules are solubilized by adding NaCl and KCl salts to the raw extract, then separated and eliminated from the phospholipid extracts to obtain a raw solid which can be stored at -20° C: in particular, the first partially purified solid phospholipid extract obtained in step h. of the intermediate purification, which can be stored at -20°C, allows the process to be possibly interrupted at the end of said step h., of the intermediate purification and carrying out the final purification at a later time
   e'. cooling to 0°C and solubilizing the wet raw extract deriving from step d. by adding NaCl and KCl, preferably in a quantity equal to 1-2 grams (per type of salt)/Kg of initial ground product obtained in step a. and in a ratio of 1:1 w/w, stirring for at least 30 minutes;
   e". settling the extract and separating the subnatant by filtering it through a filter with a filtration degree of 1µm, preferably in polypropylene, eliminating the supernatant;
   f. adding purified water to the subnatant of step e". for at least 10% of its volume, leaving it under stirring for at least 30 minutes, settling for at least 60 minutes, separating the subnatant and eliminating the supernatant;
   g'. lowering the temperature to 0°C: adding acetone to the extract from step f., preferably 1L/L of extract, and CaCl₂ dihydrate, preferably 1-2 grams/L of extract, stirring and settling for at least 30 minutes, removing the supernatant;
   g". adding acetone to the subnatant of step g'., preferably 0.1-0.2 L/L of subnatant, stirring and settling; this washing step being repeatable and carried out at 10°C;
   h. filtering the product of point g". by means of a filter press preferably with polypropylene filter/cloth (eliminating the supernatant) to obtain a first partially purified solid phospholipid extract, which can be stored at -20°C;
Final purification which comprises the following steps:
   with the following steps, the extract is made extremely fine, soluble and filterable through 0.2 µm filters which ensure bacterial sterility:
   i. solubilizing the solid phospholipid extract obtained at the end of step h. in methylene chloride or another POS, preferably in a ratio of 100-150 grams of extract/L of solvent, stirring for at least 60 minutes at 21-25°C;
   j. adding to the extract obtained from step i., the solution comprising EDTA 110-130 Grams/L, NaOH 7-9 Grams/L, KCl 27-30 Grams/L, NaCl 22-25 Grams/L, prepared in purified water and with a final pH within the range of 8.5-9, in a percentage quantity within the range of 21-24% with respect to the volume of the extract obtained at the end of step i., and subsequently ethanol in a percentage quantity of 16-20% again with respect to the volume of the extract obtained at the end of step i., stirring for at least 60 minutes and settling for at least 12 hours;
   k. separating the subnatant and eliminating the supernatant; adding purified water at 10% and ethanol at 1% of the volume of the subnatant, stirring for at least 60 minutes and settling for at least 12 hours;
   l. separating the subnatant from the supernatant (eliminated) by filtration through a filter with a filtration degree of 3µm, or alternatively 0.8µm, preferably in polypropylene, then 0.2µm, preferably in polytetrafluoroethylene, stirring and settling, separating the subnatant eliminating the supernatant;
   m. adding acetone to the sediment obtained at the end of step 1., preferably 0.5L/L of subnatant, stirring for at least 30 minutes at a temperature of 10°C, settling the phospholipid precipitate and eliminating the supernatant by siphoning; this operation can be repeated several times;
   n. filtering the product obtained at the end of step m. using a filter press preferably with a polypropylene filter/cloth (eliminating the supernatant) to obtain the purified solid phospholipid extract;
   o. treating the final extract obtained from step n. with liquid nitrogen, granulating and drying at 40°C with a vacuum of ≤ 0.5 mbars.

To avoid oxidation of the phospholipids (as known to skilled persons in the field), in the initial part of the above process, an antioxidant (for example Oxynex^{®} LM) can be conveniently added to the raw phospholipid extract.

The phospholipids were then analyzed for their identification by TLC chromatography stained with ninhydrin and/or copper sulfate as known to skilled persons in the field (Bitman J. & Wood DL.; Journal of Liquid Chromatography; 1982; 5:1155-1162 ), the tests necessary for determining their purity from biocontaminants (all of the microbiological controls required for inj ectable products according to current *Ph. Eur.)* and pyrogens *(Pyrogen Test, Ph. Eur.* 2.6.8.) were then effected, from proteins (Lowry et al.; J. Biol. Chem; 1951; 193(1):265-275) and from solvent residues (by gas chromatographic determination with the headspace technique vs reference solution, as known to skilled persons in the field).

They were then prepared in the pharmaceutical form selected with the appropriate excipient as known to skilled persons in the field.

For the uses described above, the pharmaceutical composition in injection form is preferable.

### Example 1

### Industrial preparation of PS-enriched phospholipids from swine brain

2.300 kg of swine brain were weighed and ground, the extraction step was then carried out, in particular in point b. the mixture of solvents consisting of acetone/methylene chloride/methanol was added to the ground product indicated above, in a ratio of ground product/mixture equal to 1:2.2 w/v, wherein the solvents were in a ratio of 1: 1 ,4:0.6 (v/v); the process is then continued with the subsequent steps described to arrive at the intermediate purification:
step e.: the NaCl and KCl salts were added to the wet raw material of the previous step in quantities equal to 1.5 g/Kg of initial ground product; the process is then continued with the following steps f.-h. wherein CaCl₂ dihydrate was added in a quantity equal to 1.6 g/L of extract; the process is then continued with step g. wherein 0.1L of acetone per L of subnatant produced was added, the process was then continued with step h., filtering the product by means of a filter press with a polypropylene filter obtaining the solid raw phospholipid.

### Final purification:

Step i.: the solid raw extract was solubilized with methylene chloride in a ratio of 125g/L of solvent; step j.: the solution of step j. was prepared with 122g/L of EDTA, 8g/L of NaOH, 29g/L of KCl, and 23g of NaCl/L at pH=8.5, this solution was then added to the extract obtained in step i. a. in a quantity of 23% with respect to its volume, ethanol was then added in a quantity equal to 18%; the purification process is then continued up to drying at 40°C with a vacuum of ≤ 0.5 mbars.

### Result:

Identification: the phospholipids were identified by TCL (Thin Layer Chromatography) stained with copper sulfate, it was therefore possible to compare the phospholipids present through their **RF** (Retardation Factors), i.e. based on the ratio between the distance covered by the phospholipid and the distance covered by the solvent, and they were found to be, in order (from the bottom): SFM, PC, PS, PI, PA and PE, with traces of cerebrosides and sulfatides; gangliosides absent.

In order to determine their percentage quantities and the ratio between PS and PC, the TCL was read with a photodensitometer at 450 nm:
SFM= 2.9 %
PC= 13.0 %
PS= 20.2 %
PI= 6.5 %
PA= 1.8 %
PE= 55.5 %
the PS/PC ratio = 1.55 was subsequently calculated.
The phospholipids were found to be over 90% of the phospholipid preparation.
Residual solvents: absent.
Proteins: absent.
Pyrogens and microbiological contaminants: absent

Example 1 unequivocally demonstrates how the process for the extraction and purification of phospholipids from animal brain object of the present invention determines the production of substantially pure phospholipids enriched in PS.

### Example 2:

### Pharmaceutical phospholipid composition in the treatment of the Long-Covid pathology

Case Report: S.M., 54-years old, female, contracted the Sars-Cov-2 coronavirus in April 2020, and therefore fell ill with Covid in a form of medium severity. The main symptoms were medium/severe respiratory failure, fever, headache, anosmia and ageusia, she was treated mainly with the administration of high-dose NSAIDs.

After 15 days the patient proved to be negative for nasal swabs and recovered from the pathology, however in a very short time she developed the Long-Covid disease due to the onset of a persistent feeling of fatigue/asthenia associated with "mental fog", i.e. a state of mental confusion which was such that she was unable to return to work or to daily pre-Covid occupations.

Phospholipids prepared according to the process object of the invention (Example 1), formulated on an aqueous basis with mannitol and sodium phosphate as excipients, were administered to the patient intramuscularly at a dose of 28 mg/2 ml of injectable composition, for a daily treatment of 20 days.

After about 10 days, the patient began to benefit from the phospholipid administration with a progressive decrease in both mental confusion and the feeling of fatigue/asthenia, recovering completely 20 days after the start of the treatment.

This case is demonstrative of the therapeutic effectiveness of phospholipids and the compositions containing them in the treatment of the Long-Covid pathology.

## Claims

1. A process for the extraction and purification of phospholipids enriched in phosphatidylserine PS, wherein the final PS/phosphatidylcholine PC ratio at the end of the extraction and purification steps ranges from 0.8 to 1.6, which comprises or consists of the following steps:
Extraction, which comprises the following steps:
a. grinding the animal brain;
b. adding a mixture of solvents comprising acetone and methanol to the ground product;
c. stirring and settling;
d. separating the sedimented wet raw phospholipid extract;
Intermediate purification which comprises the following steps:
e. solubilizing the wet raw phospholipid extract obtained from step d. with NaCl and KCl salts, settling, separating the subnatant by filtration;
f. washing the subnatant obtained in the previous step e. with purified water, settling and separating the subnatant;
g. adding acetone and CaCl₂ dihydrate to the subnatant of step f., stirring and settling;
h. filtering the product of step g. by means of a filter press to obtain a first partially purified solid phospholipid extract;
Final purification which comprises the following steps:
i. solubilizing the solid phospholipid extract in an organic solvent;
j. adding a solution comprising EDTA, NaOH, KCl, and NaCl having a pH of 8.5-9 to the extract of step i., stirring and settling;
k. separating the subnatant of step j., adding water and ethanol, stirring and settling;
l. separating the subnatant of step k. by filtration through a filter with a filtration degree of 3µm, or alternatively 0.8 µm, and subsequently 0.2 µm, stirring and settling;
m. washing the sediment from step l. with acetone;
n. filtering the product of step m. using a filter press to obtain the purified solid phospholipid extract;
o. treating the final extract of step n. with liquid nitrogen, granulating and vacuum-drying.

2. The extraction and purification process of PS-enriched phospholipids according to claim 1 which comprises or consists of the following steps:
Extraction which comprises the following steps:
a. grinding the animal brain, preferably its hypothalamic part and/or its cortex;
b. adding the solvent mixture consisting of acetone/methylene chloride or another polar organic solvent (POS)/methanol to the ground product of step a., in a ratio (Kg/L) (ground product) 1: (mixture) 1.5-3 w/v, preferably 1:2.2, w/v, wherein the solvents of the above mixture are in a ratio of (acetone) 1: (methylene chloride or another POS)1-2: (methanol) 0.5-1 (v/v);
c. leaving under stirring for at least 60 minutes at 30-35°C; settling the extract for at least 180 minutes at 30-35°C;
d. separating the sedimented subnatant extract of step c. by filtering it through a filter with a filtration degree of 1µm, preferably in polypropylene, eliminating the supernatant;
Intermediate purification which comprises the following steps:
e'. cooling to 0°C and solubilizing the wet raw extract deriving from step d. by adding NaCl and KCl, preferably in a quantity equal to 1-2 grams (per type of salt)/kg of initial ground product obtained in step a. and in a ratio of 1: 1 w/w, stirring for at least 30 minutes;
e". settling the extract and separating the subnatant by filtering it through a filter with a filtration degree of 1µm, preferably in polypropylene, eliminating the supernatant;
f. adding purified water to the subnatant of step e". for at least 10% of its volume, leaving it under stirring for at least 30 minutes, settling for at least 60 minutes, separating the subnatant and eliminating the supernatant;
g'. lowering the temperature to 0°C: adding acetone to the extract of step f., preferably 1L/L of extract, and CaCl₂ dihydrate, preferably 1-2 grams/L of extract, stirring and settling for at least 30 minutes, removing the supernatant;
g". adding acetone to the subnatant of step g'., preferably 0.1-0.2 L/L of subnatant, stirring and settling; this washing step being repeatable and carried out at 10°C;
h. filtering the product obtained from step g". by means of a filter press preferably with a polypropylene filter/cloth (eliminating the supernatant) to obtain a first partially purified solid phospholipid extract;
Final purification which comprises the following steps:
i. solubilizing the solid phospholipid extract obtained at the end of step h. in methylene chloride or another POS, preferably in a ratio of 100-150 grams of extract/L of solvent, stirring for at least 60 minutes at 21-25°C;
j. adding to the extract obtained from step i., the solution comprising EDTA 110-130 Grams/L, NaOH 7-9 Grams/L, KCl 27-30 Grams/L, NaCl 22-25 Grams/L, prepared in purified water and with a final pH within the range of 8.5-9, in a percentage quantity within the range of 21-24% with respect to the volume of the extract obtained at the end of step i., and subsequently ethanol in a percentage quantity of 16-20% again with respect to the volume of the extract obtained at the end of step i., stirring for at least 60 minutes and settling for at least 12 hours;
k. separating the subnatant and eliminating the supernatant; adding purified water at 10% and ethanol at 1% of the volume of the subnatant, stirring for at least 60 minutes and settling for at least 12 hours;
l. separating the subnatant from the supernatant (eliminated) by filtration through a filter with a filtration degree of 3µm, or alternatively 0.8 µm, preferably in polypropylene, then 0.2µm, preferably in polytetrafluoroethylene, stirring and settling, separating the subnatant eliminating the supernatant;
m. adding acetone to the sediment obtained at the end of step 1, preferably 0.5L/L of subnatant, stirring for at least 30 minutes at a temperature of 10°C, settling the phospholipid precipitate and eliminating the supernatant by siphoning; this operation can be repeated several times;
n. filtering the product obtained at the end of step m. using a filter press preferably with a polypropylene filter/cloth (eliminating the supernatant) to obtain the purified solid phospholipid extract;
o. treating the final extract obtained from step n. with liquid nitrogen, granulating and drying at 40°C with a vacuum of ≤ 0.5 mbars.

3. The extraction and purification process of PS-enriched phospholipids according to any of claims 1-2, which does not comprise the use of chloroform and a purification step by silica-gel chromatography.

4. The extraction and purification process of PS-enriched phospholipids according to one or more of claims 1-3, wherein the animal brain processed in step a. is swine brain.

5. PS-enriched phospholipids obtained according to the extraction and purification process according to one or more of the previous claims 1-3, wherein the final PS/PC ratio is in the range from 0.8 to 1.6.

6. Pharmaceutical compositions comprising or consisting of PS-enriched phospholipids according to claim 5, for use in the treatment of the pathology defined as "Long-Covid".

## Patentansprüche

1. Verfahren zur Extraktion und Reinigung von Phospholipiden, die an Phosphatidylserin PS angereichert sind, wobei das endgültige PS/Phosphatidylcholin PC-Verhältnis am Ende der Extraktions- und Reinigungsschritte im Bereich von 0,8 bis 1,6 liegt, das die folgenden Schritte umfasst oder daraus besteht:
Extraktion, die die folgenden Schritte umfasst:
a. Zermahlen des tierischen Gehirns;
b. Zugeben eines Gemisches von Lösungsmitteln, das Aceton und Methanol umfasst, zum gemahlenen Produkt;
c. Rühren und Absetzen;
d. Abtrennen des sedimentierten nassen Rohphospholipidextrakts;
Zwischenreinigung, die die folgenden Schritte umfasst:
e. Solubilisieren des aus Schritt d. erhaltenen nassen Rohphospholipidextrakts mit NaCl- und KCl-Salzen, Absetzen, Abtrennen des Subnatanten durch Filtration;
f. Waschen des im vorherigen Schritt e. erhaltenen Subnatanten mit gereinigtem Wasser, Absetzen und Abtrennen des Subnatanten;
g. Zugeben von Aceton und CaCl₂-Dihydrat zu dem Subnatanten von Schritt f., Rühren und Absetzen;
h. Filtrieren des Produkts von Schritt g. mittels einer Filterpresse, um einen ersten teilweise gereinigten festen Phospholipidextrakt zu erhalten;
Endreinigung, die die folgenden Schritte umfasst:
i. Solubilisieren des festen Phospholipidextrakts in einem organischen Lösungsmittel;
j. Zugeben einer Lösung, umfassend EDTA, NaOH, KCl und NaCl mit einem pH-Wert von 8,5-9 zum Extrakt von Schritt i., Rühren und Absetzen;
k. Abtrennen des Subnatanten von Schritt j., Zugeben von Wasser und Ethanol, Rühren und Absetzen;
1. Abtrennen des Subnatanten von Schritt k. durch Filtration über einen Filter mit einem Filtrationsgrad von 3 µm oder alternativ 0,8 µm und anschließend 0,2 µm, Rühren und Absetzen;
m. Waschen des Sediments aus Schritt 1. mit Aceton;
n. Filtrieren des Produkts von Schritt m. unter Verwendung einer Filterpresse, um den gereinigten festen Phospholipidextrakt zu erhalten;
o. Behandeln des endgültigen Extrakts von Schritt n. mit flüssigem Stickstoff, Granulieren und Vakuumtrocknen.

2. Extraktions- und Reinigungsverfahren von PS-angereicherten Phospholipiden nach Anspruch 1, das die folgenden Schritte umfasst oder daraus besteht:
Extraktion, die die folgenden Schritte umfasst:
a. Zermahlen des tierischen Gehirns, vorzugsweise seines hypothalamischen Teils und/oder seiner Hirnrinde;
b. Zugeben des Lösungsmittelgemisches, bestehend aus Aceton/Methylenchlorid oder einem anderen polaren organischen Lösungsmittel (POS)/Methanol zu dem gemahlenen Produkt von Schritt a., in einem Verhältnis (Kg/L) (gemahlenes Produkt) 1: (Gemisch) 1,5-3 G/V, vorzugsweise 1:2,2, G/V, wobei die Lösungsmittel des obigen Gemisches in einem Verhältnis von (Aceton): (Methylenchlorid oder einem anderen POS)1-2: (Methanol) 0,5-1 (V/V) stehen;
c. Lassen mindestens 60 Minuten bei 30-35 °C unter Rühren; Absetzen des Extrakts für mindestens 180 Minuten bei 30-35 °C;
d. Abtrennen des sedimentierten subnatanten Extrakts von Schritt c. durch Filtrieren über einen Filter mit einem Filtrationsgrad von 1 µm, vorzugsweise in Polypropylen, wobei der Überstand eliminiert wird;
Zwischenreinigung, die die folgenden Schritte umfasst:
e'. Abkühlen auf 0 °C und Solubilisieren des aus Schritt d. stammenden nassen Rohextrakts durch Zugeben von NaCl und KCl, vorzugsweise in einer Menge gleich 1-2 Gramm (pro Salzart)/kg des in Schritt a. erhaltenen gemahlenen Ausgangsprodukts und im Verhältnis 1:1 G/G, Rühren für mindestens 30 Minuten;
e". Absetzen des Extrakts und Abtrennen des Subnatanten durch Filtrieren über einen Filter mit einem Filtrationsgrad von 1 µm, vorzugsweise in Polypropylen, wobei der Überstand eliminiert wird;
f. Zugeben von gereinigtem Wasser zu dem Subnatanten von Schritt e" für mindestens 10 % seines Volumens, Lassen für mindestens 30 Minuten unter Rühren, Absetzen für mindestens 60 Minuten, Abtrennen des Subnatanten und Eliminieren des Überstandes;
g'. Absenken der Temperatur auf 0 °C: Zugeben von Aceton zu dem Extrakt von Schritt f., vorzugsweise 1 L/L Extrakt, und CaCl₂-Dihydrat, vorzugsweise 1-2 g/L Extrakt, Rühren und Absetzen für mindestens 30 Minuten, Entfernen des Überstands;
g". Zugeben von Aceton zu dem Subnatanten von Schritt g'., vorzugsweise 0,1-0,2 L/L Subnatant, Rühren und Absetzen; dieser Waschschritt ist wiederholbar und wird bei 10 °C durchgeführt;
h. Filtrieren des aus Schritt g". erhaltenen Produkts mittels einer Filterpresse, vorzugsweise mit einem Polypropylenfilter/-tuch (wobei der Überstand eliminiert wird), um einen ersten teilweise gereinigten festen Phospholipidextrakt zu erhalten;
Endreinigung, die die folgenden Schritte umfasst:
i. Solubilisieren des am Ende von Schritt h. erhaltenen festen Phospholipidextrakts in Methylenchlorid oder einem anderen POS, vorzugsweise in einem Verhältnis von 100-150 g Extrakt/L Lösungsmittel, Rühren für mindestens 60 Minuten lang bei 21-25 °C;
j. Zugeben zu dem aus Schritt i. erhaltenen Extrakt, die Lösung, umfassend EDTA 110-130 Gramm/L, NaOH 7-9 Gramm/L, KCl 27-30 Gramm/L, NaCl 22-25 Gramm/L, hergestellt in gereinigtem Wasser und mit einem End-pH-Wert im Bereich von 8,5-9, in einer prozentualen Menge im Bereich von 21-24%, bezogen auf das Volumen des am Ende von Schritt i. erhaltenen Extrakts, und anschließend Ethanol in einer prozentualen Menge von 16-20%, wieder bezogen auf das Volumen des am Ende von Schritt i. erhaltenen Extrakts, Rühren für mindestens 60 Minuten und Absetzen für mindestens 12 Stunden;
k. Abtrennen des Subnatanten und Eliminieren des Überstands; Zugeben von gereinigtem Wasser in Höhe von 10 % und Ethanol in Höhe von 1 % des Volumens des Subnatanten, Rühren für mindestens 60 Minuten und Absetzen für mindestens 12 Stunden;
l. Abtrennen des Subnatanten vom Überstand (eliminiert) durch Filtration über einen Filter mit einem Filtrationsgrad von 3 µm, oder alternativ 0,8 µm, vorzugsweise in Polypropylen, dann 0,2 µm, vorzugsweise in Polytetrafluorethylen, Rühren und Absetzen, Abtrennen des Subnatanten und Eliminieren des Überstands;
m. Zugeben von Aceton zu dem am Ende von Schritt 1 erhaltenen Sediment, vorzugsweise 0.5L/L des Subnatanten, Rühren für mindestens 30 Minuten bei einer Temperatur von 10 °C, Absetzen des Phospholipidniederschlags und Eliminieren des Überstands durch Absaugen; dieser Vorgang kann mehrmals wiederholt werden;
n. Filtrieren des am Ende von Schritt m. erhaltenen Produkts unter Verwendung einer Filterpresse, vorzugsweise mit einem Polypropylenfilter/-tuch (wobei der Überstand eliminiert wird), um den gereinigten festen Phospholipidextrakt zu erhalten;
o. Behandeln des aus Schritt n. erhaltenen endgültigen Extrakts mit flüssigem Stickstoff, Granulieren und Trocknen bei 40 °C mit einem Vakuum von :S 0,5 mbar.

3. Extraktions- und Reinigungsverfahren von PS-angereicherten Phospholipiden nach einem der Ansprüche 1-2, das nicht die Verwendung von Chloroform und einen Reinigungsschritt durch Kieselgelchromatographie umfasst.

4. Extraktions- und Reinigungsverfahren von PS-angereicherten Phospholipiden nach einem oder mehreren der Ansprüche 1-3, wobei das in Schritt a. verarbeitete Tiergehirn Schweinegehirn ist.

5. PS-angereicherte Phospholipide, erhalten nach dem Extraktions- und Reinigungsverfahren nach einem oder mehreren der vorhergehenden Ansprüche 1-3, wobei das endgültige PS/PC-Verhältnis im Bereich von 0,8 bis 1,6 liegt.

6. Pharmazeutische Zusammensetzungen, die PS-angereicherte Phospholipide nach Anspruch 5 umfassen oder daraus bestehen, zur Verwendung bei der Behandlung der als "Long-Covid" definierten Pathologie.

## Revendications

1. Procédé d'extraction et de purification de phospholipides enrichis en phosphatidylsérine PS, dans lequel le rapport final PS/phosphatidylcholine PC à la fin des étapes d'extraction et de purification est compris entre 0,8 et 1,6, qui comprend ou consiste en les étapes suivantes:
l'extraction, qui comprend les étapes suivantes:
a. le broyage du cerveau animal;
b. l'ajout au produit broyé d'un mélange de solvants comprenant de l'acétone et du méthanol;
c. l'agitation et la décantation;
d. la séparation de l'extrait de phospholipides brut humide sédimenté;
la purification intermédiaire qui comprend les étapes suivantes :
e. la solubilisation de l'extrait de phospholipides brut humide obtenu à l'étape d. avec des sels de NaCl et de KCl, la décantation, la séparation du surnageant par filtration ;
f. le lavage du surnageant obtenu à l'étape e. précédente avec de l'eau purifiée, la décantation et la séparation du surnageant ;
g. l'ajout d'acétone et de CaCl₂ dihydraté au surnageant de l'étape f., l'agitation et la décantation ;
h. la filtration du produit de l'étape g. au moyen d'un filtre-presse pour obtenir un premier extrait de phospholipides solide partiellement purifié ;
la purification finale qui comprend les étapes suivantes :
i. la solubilisation de l'extrait de phospholipides solide dans un solvant organique ;
j. l'ajout à l'extrait de l'étape i. d'une solution comprenant de l'EDTA, du NaOH, du KCl et du NaCl ayant un pH compris entre 8,5 et 9, l'agitation et la décantation ;
k. la séparation du surnageant de l'étape j., l'ajout d'eau et d'éthanol, l'agitation et la décantation ;
l. la séparation du surnageant de l'étape k. par filtration à travers un filtre d'un degré de filtration de 3 µm, ou alternativement de 0,8 µm, et ensuite de 0,2 µm, l'agitation et la décantation ;
m. le lavage du sédiment de l'étape 1 avec de l'acétone ;
n. la filtration du produit de l'étape m. à l'aide d'un filtre-presse pour obtenir l'extrait de phospholipides solide purifié ;
o. le traitement de l'extrait final de l'étape n. avec de l'azote liquide, la granulation et le séchage sous vide.

2. Procédé d'extraction et de purification des phospholipides enrichis en PS selon la revendication 1 qui comprend ou consiste en les étapes suivantes :
l'extraction qui comprend les étapes suivantes :
a. le broyage du cerveau animal, de préférence sa partie hypothalamique et/ou son cortex ;
b. l'ajout du mélange de solvants composé d'acétone/de chlorure de méthylène ou d'autre solvant organique polaire (POS)/de méthanol au produit broyé de l'étape a., dans un rapport (Kg/L) (produit broyé) 1 : (mélange) 1,5-3 w/v, de préférence 1:2,2, w/v, dans lequel les solvants du mélange ci-dessus sont dans un rapport de (acétone)! : (chlorure de méthylène ou autre POS)1-2 : (méthanol) 0,5-1 (v/v) ;
c. le maintien sous agitation pendant au moins 60 minutes à 30-35°C ; la décantation de l'extrait pendant au moins 180 minutes à 30-35°C ;
d. la séparation de l'extrait de couche inférieure sédimenté de l'étape c. en le filtrant à travers un filtre d'un degré de filtration de 1 µm, de préférence en polypropylène, en éliminant le surnageant ;
la purification intermédiaire qui comprend les étapes suivantes :
e'. le refroidissement à 0°C et la solubilisation de l'extrait brut humide obtenu à l'étape d. en ajoutant du NaCl et du KCl, de préférence en quantité égale à 1-2 grammes (par type de sel)/kg de produit initial broyé obtenu à l'étape a. et dans un rapport de 1:1 w/w, en agitant pendant au moins 30 minutes ;
e". la décantation de l'extrait et la séparation de la couche inférieure par filtration à travers un filtre d'un degré de filtration de 1 µm, de préférence en polypropylène, en éliminant le surnageant ;
f. l'ajout d'eau purifiée à la couche inférieure de l'étape e". pour au moins 10 % de son volume, le maintien sous agitation pendant au moins 30 minutes, la décantation pendant au moins 60 minutes, la séparation de la couche inférieure et l'élimination du surnageant ;
g'. l'abaissement de la température à 0°C : l'ajout d'acétone à l'extrait de l'étape f., de préférence 1L/L d'extrait, et de CaCl₂ dihydraté, de préférence 1-2 grammes/L d'extrait,
l'agitation et la décantation pendant au moins 30 minutes, l'élimination du surnageant ;
g". l'ajout d'acétone à la couche inférieure de l'étape g'., de préférence 0,1-0,2 L/L de couche inférieure, l'agitation et la décantation ; cette étape de lavage peut être répétée et est effectuée à 10°C ;
h. la filtration du produit obtenu à l'étape g". au moyen d'un filtre-presse de préférence avec un filtre/toile en polypropylène (en éliminant le surnageant) pour obtenir un premier extrait de phospholipides solide partiellement purifié ;
la purification finale qui comprend les étapes suivantes :
i. la solubilisation de l'extrait de phospholipides solide obtenu à l'issue de l'étape h. dans du chlorure de méthylène ou un autre POS, de préférence dans un rapport de 100-150 grammes d'extrait/L de solvant, sous agitation pendant au moins 60 minutes à 21-25°C ;
j. l'ajout à l'extrait obtenu à l'étape i., de la solution comprenant EDTA 110-130 Grammes/L, NaOH 7-9 Grammes/L, KCl 27-30 Grammes/L, NaCl 22-25 Grammes/L, préparée dans de l'eau purifiée et avec un pH final compris entre 8,5 et 9, dans une quantité en pourcentage comprise entre 21 et 24 % par rapport au volume de l'extrait obtenu à la fin de l'étape i., puis de l'éthanol dans une quantité en pourcentage comprise entre 16 et 20 % par rapport au volume de l'extrait obtenu à la fin de l'étape i, l'agitation pendant au moins 60 minutes et la décantation pendant au moins 12 heures ;
k. la séparation de la couche inférieure et l'élimination du surnageant ; l'ajout d'eau purifiée à 10 % et d'éthanol à 1 % du volume de la couche inférieure, l'agitation pendant au moins 60 minutes et la décantation pendant au moins 12 heures ;
l. la séparation de la couche inférieure du surnageant (éliminé) par filtration à travers un filtre d'un degré de filtration de 3 µm, ou alternativement de 0,8 µm, de préférence en polypropylène, puis de 0,2 µm, de préférence en polytétrafluoroéthylène, l'agitation et la décantation, la séparation de la couche inférieure éliminant le surnageant ;
m. l'ajout d'acétone au sédiment obtenu à la fin de l'étape 1, de préférence 0,5L/L de la couche inférieure, l'agitation pendant au moins 30 minutes à une température de 10°C, la décantation du précipité de phospholipides et l'élimination du surnageant par siphonage ; cette opération peut être répétée plusieurs fois ;
n. la filtration du produit obtenu à l'issue de l'étape m. à l'aide d'un filtre-presse de préférence avec un filtre/toile en polypropylène (en éliminant le surnageant) pour obtenir l'extrait de phospholipide solide purifié ;
o. le traitement de l'extrait final obtenu à l'étape n. avec de l'azote liquide, la granulation et le séchage à 40°C sous un vide de :S 0,5 mbars.

3. Procédé d'extraction et de purification de phospholipides enrichis en PS selon l'une quelconque des revendications 1 à 2, ne comprenant pas l'utilisation de chloroforme et une étape de purification par chromatographie sur gel de silice.

4. _ Procédé d'extraction et de purification de phospholipides enrichis en PS selon l'une ou plusieurs des revendications 1 à 3, dans lequel le cerveau animal traité à l'étape a. est un cerveau de porc.

5. Phospholipides enrichis en PS obtenus selon le procédé d'extraction et de purification selon l'une ou plusieurs des revendications précédentes 1 à 3, dans lesquels le rapport PS/PC final est compris entre 0,8 et 1,6.

6. Compositions pharmaceutiques comprenant ou consistant en des phospholipides enrichis en PS selon la revendication 5, pour une utilisation dans le traitement de la pathologie définie comme "Covid long".
